Europäisches Patentamt

European Patent Office

Office européen des brevets

(11) Publication number: **0 377 057**
**A1**

## (12) EUROPEAN PATENT APPLICATION

(21) Application number: **88830572.9**

(22) Date of filing: **30.12.88**

(51) Int. Cl.⁵: **A61N 1/34, A61N 1/32**

(43) Date of publication of application:
**11.07.90 Bulletin 90/28**

(84) Designated Contracting States:
**AT BE CH DE ES FR GB GR IT LI LU NL SE**

(71) Applicant: **Bernardini, Leandro**
**Via di Baroncoli 10**
**Calenzano (Firenze)(IT)**

Applicant: **Bernardini, Franco**
**Via di Vittorio 35**
**Sesto Fiorentino (Firenze)(IT)**

Applicant: **Zanni, Paolo**
**Via Quintino Sella 35**
**Firenze(IT)**

(72) Inventor: **Bernardini, Leandro**
**Via di Baroncoli 10**
**Calenzano (Firenze)(IT)**
Inventor: **Bernardini, Franco**
**Via di Vittorio 35**
**Sesto Fiorentino (Firenze)(IT)**
Inventor: **Zanni, Paolo**
**Via Quintino Sella 35**
**Firenze(IT)**

(74) Representative: **Sassatelli, Franco T., Dr.**
**c/o INIP via Ruggi 5**
**I-40137 Bologna(IT)**

(54) **Hand-operated electromedical device for analgesic skin treatments.**

㊐ For the use, the device equipped with a switch (1) is gripped and brought on the part to treat and, by acting on the small regulation wheel, the operator adjustes the outlet power according to the checking sensibility. He then goes on to treatment by means of subsequent passages and/or stays by properly relating the pressure of the contact head with the skin according to the use requirements. By means of the switch (1), the current from a battery 2, through a potentiometer (3), reaches a swinging circuit (4) which, on four lines (5, 6, 7 and 8), feeds an induction coil (9) fitted with a primary (10) and four secondary (11, 12, 13 and 14) circuits, which on a head (15) feed a set of terminal electrodes (16). The device can be used by the interested person without the product "GEL" on the electrodes.

Fig 1

## Hand-operated electromedical device for analgesic skin treatments.

The invention refers to a small-sized electric-analgesical device, with an ideally longitudinal body to be seized in the hand and with an epidermical contact head, fitted with a set of electrodes, to be used by the occasional person interested in the treatment by means of essential sequences with mnemonic checking and without usign products facilitating the electrode contact on the skin. The said features enable the use of this pocket type medical device as a component of the conventional outfit for private users. A new conception, extremely cheap small size means thus results which enables continuous and prompt intervention.

It is well known that the levels of the central and peripheral nervous centres are stimulated by means of electrical impulse emission through the skin. Consequently, by means of electrical skin stimulus, it is possible to soothe the ache of an inside organ, which is a direct electric conductivity with the epidermic plates interested in the treatment, by again equilibrating its energetical charge. Therefore, a reduction or the elimination of a pain of spontaneous pathological origin of reflected type, and particularly in consequence of traumas, can be reached.

At present, such treatment can be carried out exclusively by the specialist physician or physiatre in a laboratory by means of devices generally using an oscillator which feeds some high tension coils connected with mobile contacts with a set of electrodes. These contacts are directly fitted on the skin of the patient lying on a couch. In order to ensure a useful contact for the penetration of the electric impulses, a salt base "GEL" is laid on the electrodes, and these ones, in their turn, necessarily must be anchored on the skin by means of adhesive plasters. Should the device be not well fitted, the patient feels a tiresome smart due to the consequent surface burn, and the electrical impulses do not penetrate into the skin.

The invention device avoids all these inconveniences by adopting a pocket type apparatus to be directly used by the occasional operator by means of an elementary methodology and proceeding according to sequences with mnemonic checking. To carry out the therapy, the user grips the apparatus - ideally longitudinal cylindrical in its initial part - and brings the epidermical contact head fitted on its other end on the skin zone to be treated, then by activating the switch 1 with the same hand. In running prosecution, by acting on the small regulation wheel on the same switch, the user suits the outlet power according to the checking sensibility: the general one and the other one of the organ interested in the treatment. The therapy can be carried out by performing subsequent passages and/or stops by properly relating the pressure of the contact head on the skin according to the necessity of performing a good contact between this one and the electrodes. The necessity is consequently avoided of using "GE" on these electrodes as well as the relevant clamping on the skin by means of adhesive plasters.

With switch 1, from battery 2, the current passes, through the potentiometer 3, to the oscillating circuit 4 which, on lines 5, 6, 7 and 8, feeds the induction coil 9 fitted with a primary 10 and four secondary circuits 11, 12, 13 and 14 that feed the set of terminal electrodes 16 on head 15. By changing the kind of electrical circuit, it is possible to arrange devices in order to generate different wave shapes, thus allowing to carry out devices for different pathologies.

The device shows the coating component 17 with groove 18, for fitting the inside organs and, in free coupling, in the lower opening 19, the head 15 with getting out electrodes 16 connected with bobbin 9 in A.T. by means of connections 11, 12, 13 and 14. The said parts are then coupled by means of resining which connects and isolates them thus avoiding electrical discharges. By means of connections 5 and 8, printed circuit 4 in M.T. is reached oscillating and connected with the potentiometer 3 and, through switch 1, to the feeding battery 2. The presence of channel 18 in enveloping conponent 17 enables the laying of components 2, 4 and 9 on a line, as well as of relevant connections which causes a composition essentiality and an easy intervention in case of reinstatements.

For completing the containment coating, plug 20 is engaged and blocked by a 45° rotation and thus a counterposed pair of reliefs 21 on projections 22 of component 11 is engaged. Cover 23 is then fitted by means of an initial approaching contraction of the pair of tangs 24 of component 11 and their insertion into its seat 25. For checking the device working, a warning light 25 is foreseen which draws tension by means of connections 26 and 27.

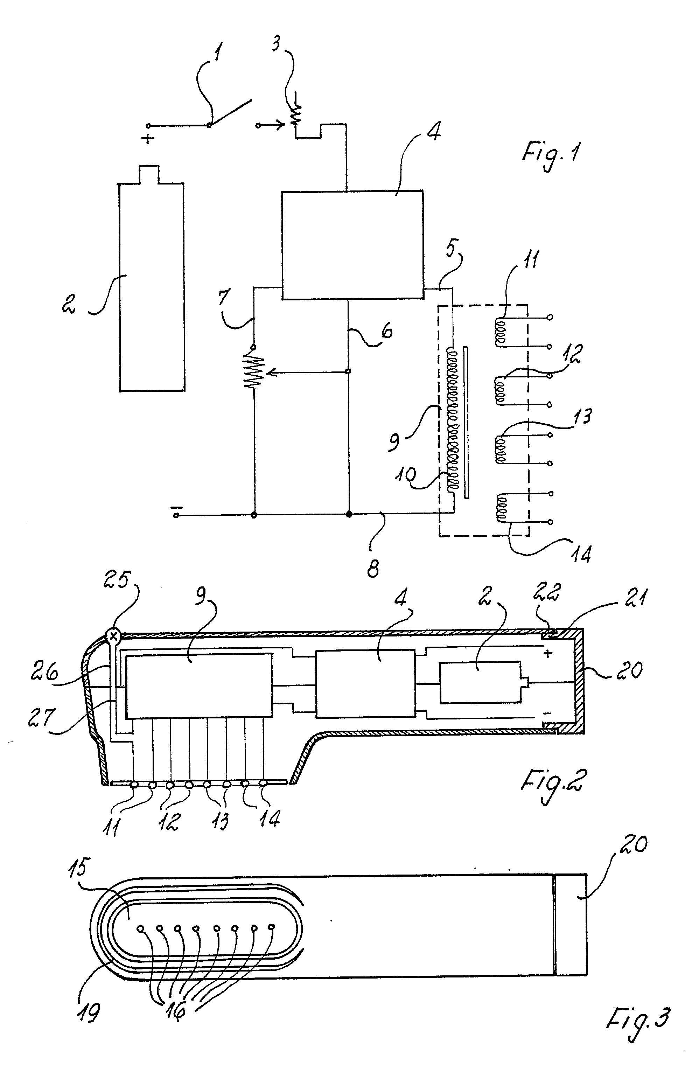

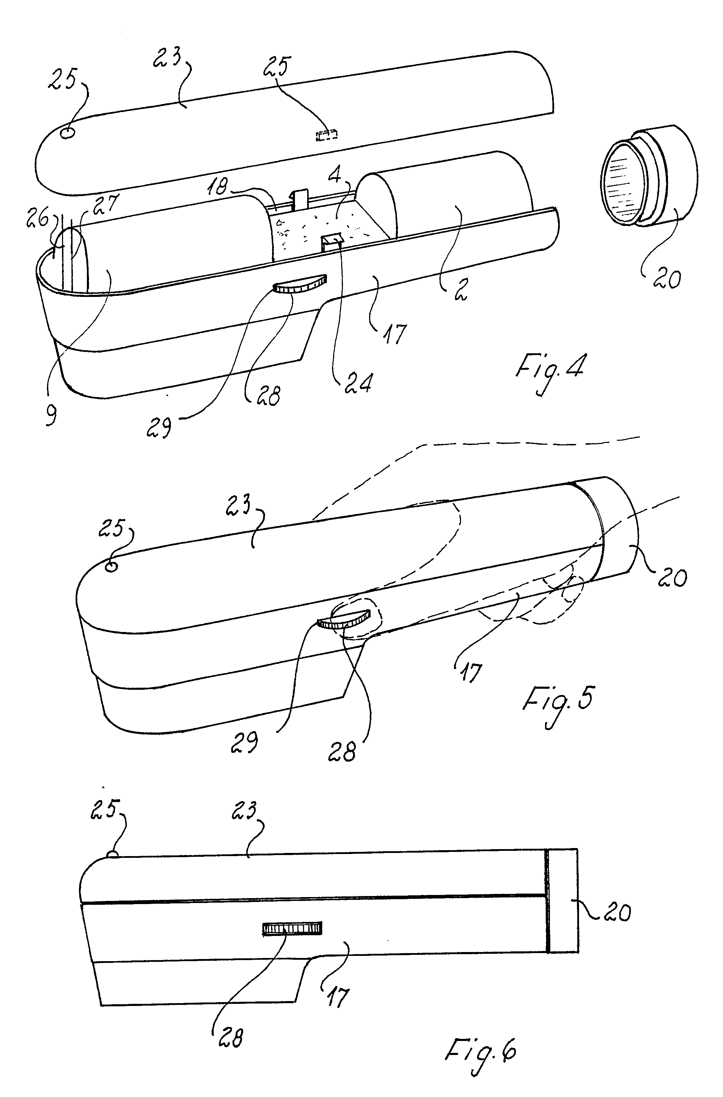

An execution form is illustrated in an indicative way by the drawings of tables 1 and 2. With reference fo table 1, fig. 1 is the wiring diagram of the device. Fig. 2 is its longitudinal section while fig. 3 is the view from below. In table 2, fig. 4 is the perspective section of the three components 17, 20 and 23 with battery 2, the swinging circuit 4 and coil 9 fitted on line in canal 18 of the basecomponent 17. Fig. 5 is the perspective view of the device with dashed hand which grips it. It ca, be noted that the user activates the small wheel 28 of switch 1 coming out from the lateral slit 29 with a hand finger. Fig. 6 is the side view of the same device.

In the executions, the body of the coating can be manufactured in different ways as far as the shape, the materials and the different integrating components and/or connection are concerned. The inside organs can vary too as well as be replaced and/or integrated in different ways according to the use requirements.

## Claims

1) Hand-operated electromedical device for analgesic skin treatments, characterized by the fact that, for using it, the device is gripped in its initial longitudinal coating part, the contact head is brought on the skin and the switch (1) activated with the same hand by regulating its issue power by means of the small wheel (28)accoring to the checking sensibility. The therapy can be executed by means of subsequent passages and/or stayings on the part to treat by relating the pressure of the head to the exigency of keeping a good contact between the electrodes and the skin. The switch (1) activation lets the current pass from the battery (2), through the potentiometer (3), to the oscillating circuit (4) which, on four lines (5, 6, 7 and 8), feeds the induction coil (9) fitted with one primary (10) and four secondary (11, 12, 13 and 14) circuits feeding the set of end electrodes (16) on the head (15).

2) Hand-operated electromedical device for analgesic skin treatments, as per claim 1), characterized by the fact that, by changing the kind of the electrical circuit in the device, it is possible to prepare in advance devices of the same type which generate different forms of emitting waves. This enables to manufacture devices to be used singularly for different pathologies.

3) Hand-operated electromedical device for analgesic skin treatments, as per claim 1), characterized by the fact that it shows a coating c omponent (17) with a fitting canal (18) for the inside organs and with a small head (15), inserted in free coupling in the lower opening (19), with emerging electrodes (16) connected with the coil (9) in A.T. by means of connections (11, 12, 13 and 14). These parts are then coupled by means of a resin layer which connects and isolates the above organs avoiding electrical discharges. By means of two connections (5 and 8), the swinging printed circuit (4) is reached in M.T. connected with the potentiomerter (3) and, through switch (1), with the feeding battery (2).

4) Hand-operated electromedical device for analgesic skin treatments, as per claim 1), characterized by the fact that the presence of a groove (18), in the coating component, allows the line disposition of several components (2, 4 and 9) and of the relevant connections determining a composition essentiality and facilitates intervention in the case of reinstatements.

5) Hand-operated electromedical device for analgesic skin treatments, as per claim 1), characterized by the fact that for completing the containing coating, a plug (20) is engaged and blocked by a 45° rotation, thus determining the insertion of the counterposed pair of reliefs (21) on the two prominences (22) of the coating component (17). Cover (23) is then fitted by means of an initial approaching contraction of the tang pair (24) of the coating component (17), as well as their insertion in its seats (25).

6) Hand-operated electromedical device for analgesic skin treatments, as per claim 1), characterized by the fact that, for checking the device working, a warning light (25) has been foreseen which draws tension by means of two connections (26 and 27).

7) Hand-operated electromedical device for analgesic skin treatments, as per claim 1), characterized by the fact that the user activates the small wheel (28) of the switch (1) in the slit (29) and thus adjustes the outlet power.

Fig.1

Fig.2

Fig.3

Fig. 4

Fig. 5

Fig. 6

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-4 763 657 (CHEN) <br> * Column 1, line 66 - column 3, line 42 * | 1-4 | A 61 N 1/34 <br> A 61 N 1/32 |
| X | US-A-4 033 356 (HARA) <br> * Column 3, line 17 - column 4, line 66 * | 1-4,7 | |
| X | US-A-4 175 551 (D'HAENENS) <br> * Column 2, line 36 - column 4, line 51 * | 1,3,6 | |
| A | DE-C- 538 579 (ERBACHER) <br> * Whole document * | 1,3,7 | |
| A | FR-A-2 087 227 (LE CLERC) <br> * Page 2, lines 1-39; page 5, lines 11-35 * | 1,3,4,5 ,6,7 | |

TECHNICAL FIELDS
SEARCHED (Int. Cl.5)

A 61 N
A 61 H

The present search report has been drawn up for all claims

| Place of search | Date of completion of the search | Examiner |
|---|---|---|
| THE HAGUE | 30-08-1989 | LEMERCIER D.L.L. |

EPO FORM 1503 03.82 (P0401)